(19) **Europäisches Patentamt**

**European Patent Office**

**Office européen des brevets**

(11) **EP 0 695 174 B1**

(12) **EUROPEAN PATENT SPECIFICATION**

(45) Date of publication and mention
of the grant of the patent:
**19.03.2003 Bulletin 2003/12**

(51) Int Cl.$^7$: **A61K 9/48**, A61K 9/00

(21) Application number: **93909607.9**

(86) International application number:
**PCT/US93/03732**

(22) Date of filing: **20.04.1993**

(87) International publication number:
**WO 94/023705 (27.10.1994 Gazette 1994/24)**

(54) **MULTI-STAGE DRUG DELIVERY SYSTEM**

MEHRSTUFIGES ARZNEISTOFFABGABESYSTEM

SYSTEME D'APPORT MEDICAMENTEUX EN PLUSIEURS ETAPES

(84) Designated Contracting States:
**AT BE CH DE DK ES FR GB GR IE IT LI LU MC NL
PT SE**

(43) Date of publication of application:
**07.02.1996 Bulletin 1996/06**

(73) Proprietor: **TSRL, Inc.
Ann Arbor, MI 48108 (US)**

(72) Inventors:
• **AMIDON, Gordon L.
Ann Arbor, MI 48107 (US)**
• **LEESMAN, Glen D.
Ann Arbor, MI 48105 (US)**
• **SHERMAN, Lizbeth B.
Ann Arbor, MI 48108 (US)**

(74) Representative: **Cawdell, Karen Teresa et al
Urquhart-Dykes & Lord
Three Trinity Court
21-27 Newport Road
Cardiff, CF24 0AA (GB)**

(56) References cited:
**EP-A- 0 384 642        EP-A- 0 384 646
US-A- 4 865 849**

Remarks:
The file contains technical information submitted
after the application was filed and not included in this
specification

## Description

## TECHNICAL FIELD

This invention relates generally to drug delivery systems.

## BACKGROUND OF THE INVENTION

It has been recognized that there is a need for a drug delivery system which yields an increase in the oral dosing interval of drugs exhibiting presystemic loss metabolism while simultaneously maintaining bioavailability equivalent to the immediate release dosage form. Such drugs would otherwise either require short interval dosing, such as periodic oral dosing having short periods between each oral dosing.

Various drug delivery systems, commonly referred to as time released systems, have attempted to solve this problem by continuously releasing amounts of the drug throughout the travel of the drug through the digestive track. For example, the United States patent 4,773,907 to Urquhart et al, issued September 27, 1988, discloses a delivery system comprising a capsule containing dosage forms comprising a semipermeable wall surrounding a compartment containing drug. A passageway through the semipermeable wall releases drug from the dosage form to the environment. The U.S. Patent 4,777,049 to Magruder et al, issued October 11, 1988, discloses an osmotic delivery system. The system provides a device including a wall which can be a laminate comprising a semipermeable lamina and lamina arrangement with a microporous lamina. The lamina provides micropaths for emitting external fluid into the osmotic device. The device includes an opening having an erodible element, such as a gelatin plug that erodes and forms an osmotic passageway in the environment of use. Within the device is a modulating agent in nonequilibrium proportions. Upon the influx of fluid into the device, there is co-solublization of a useful agent which is then released from the device. Thusly, co-solublization of a modulating agent and a useful agent controls the release of the useful agent and results in the delayed release of the useful agent resulting from a reduction of the concentration of the modulating agent. This results in an osmotic system and a method of preprogramming to a desired time of release, a delayed release or a delayed pulsed release of agent. However, the delayed pulse of release is over a base line release and not a true pulse release from a zero base line.

The U.S. Patent 4,783,337 to Wong et al, issued November 8, 1988, discloses an osmotic system comprising a wall which is at least in part a semipermeable material that surrounds a compartment. An osmotic composition, or several osmotic compositions are contained within the compartment defined by the wall and a passageway in the wall connects the first composition with the exterior of the system. The first composition causes imbibition of fluid which results in the delivery of the suspension or solution through the aforementioned passageway. This can end up being a multi-chamber device.

A further drug delivery system is disclosed in EP 0384 642. The drug delivery system disclosed comprises a container and a plug for plugging the container; the contents of the container being a water-swellable material and an active ingredient. The active ingredient is released from the container by action of the water swellable material swelling thereby forcing the plug away from the container, the active ingredient subsequently being released.

The aforementioned patents do not result in a truly pulsatile release. Pulsatile release, as used herein, implies an initial first release followed by a period of time where there is absolutely no release. Then, after the predetermined period, there is a true pulse release. Unlike prior art systems, it is desirable to provide a drug delivery system for non-linear presystemic loss drugs which will release fractions of the total dose at specified sites and time in the gastro-intestinal track so that bioavailability will not be compromised by the decreased release rate of conventionally controlled or sustained release dosage forms.

There are several advantages to a true pulsatile delivery system in extending the dosing interval. For those drugs which are first pass metabolized, an increase in delivery rate to the portal system results in a decrease in metabolism. For those drugs exhibiting non-linear prehepatic metabolism a larger fraction of drug will escape metabolism and therefore be available. For those drugs with incomplete absorption due to low permeability, poor solubility or in which case the absorption rate limited by rate of dissolution, enhancers can be added to increase the bioavailability. The pulse time and release rate can be programmed to match the immediate release dosage form profile. The pulse time and release rate from pulsatile delivery can be more reproducible than the immediate release dosage form which relies on patient compliance and rate of gastric emptying for input of drug to the site of absorption, that being the small intestine. The result is a decreased variability in plasma level time curves. The clinical efficacy of a pulsatile delivery system can be established to provide equivalent bioavailability to the conventional dosage form. Accordingly, patient compliance is increased through the use of a reduced and/or simpler dosing schedule. The pharmacodynamics of the pulsatile system can be made to match the established immediate release dosage. Thereby, the metabolic rates equivalent to that obtained from an approved dosing schedule can be obtained, hence no unusual accumulation of metabolites or altered metabolic profile results. The pulse delay and amount being pulsed are programmable to a variety of dosing schedules such that allowance for circadian rhythms is possible in order to optimize the pharmacodynamic response throughout the day. Finally, the optimal dosing schedule for two or more drugs, tailored to their individual pharmacokinetic and pharmacodynamic properties, can be optimized using this technology. The present invention provides an improved means of providing a pulsed dose or doses which are capable of providing all of the aforementioned advantages.

## SUMMARY OF THE INVENTION

In accordance with the present invention, there is provided a drug delivery system as defined in claim 1. Further preferred features are defined in subsidiary claims.

The present invention further provides a method of making a drug delivery system, the method including the steps of filling a first capsule half with drug and a reactive agent, the capsule being water permeable. The capsule half is plugged and a water permeable film is disposed over the capsule half and plug. A second capsule half is filled with drug and an open end thereof is releasably mounted over the plugged end of the first capsule half.

## FIGURES IN THE DRAWINGS

Other advantages of the present invention will be readily appreciated as the same becomes better understood by reference to the following detailed description when considered in connection with the accompanying drawings wherein:

Figure 1A is cross sectional view of a drug delivery system made in accordance with the present invention;

Figure 1B is a cross sectional view of a multichamber drug delivery system made in accordance with the present invention;

Figure 2 shows the steps of manufacturing the drug delivery system of the present invention;

Figure 3 schematically shows the steps of drug release from the drug delivery system of the present invention over time;

Figure 4 is a graph showing the percent release of drug from drug delivery systems made in accordance with the present invention over time;

Figure 5 is a graph showing the average pulse time as function of percent coating;

Figure 6 shows graphically the results of of water uptake studies on capsules made in accordance with the present invention showing percent weight gain over time;

Figure 7 shows the results of water uptake studies graphically on lactose filled capsules having different coating weights;

Figure 8 shows the results of water uptake studies of capsules containing lactose/sorbital therein, the capsules having two different weight coating thereon;

Figures 9A and B are chromatograms from blank samples and actual samples from dog studies discussed below;

Figure 10 shows a plot of chromatographic peak height ratio versus concentration; and

Figures 11A and B show two graphs illustrating the pulsatile release of drug in vivo.

## DETAILED DESCRIPTION OF THE INVENTION

A drug delivery system constructed in accordance with the present invention is generally shown at 10 in the Figures. This system generally includes a first container in the form of a capsule half 12 and a second container in the form of a mating capsule half 14. The first capsule half 12 includes an inner chamber 16 for containing a drug 18 therein. Of course, the shape of and size of the capsule half can be varied in accordance with the art.

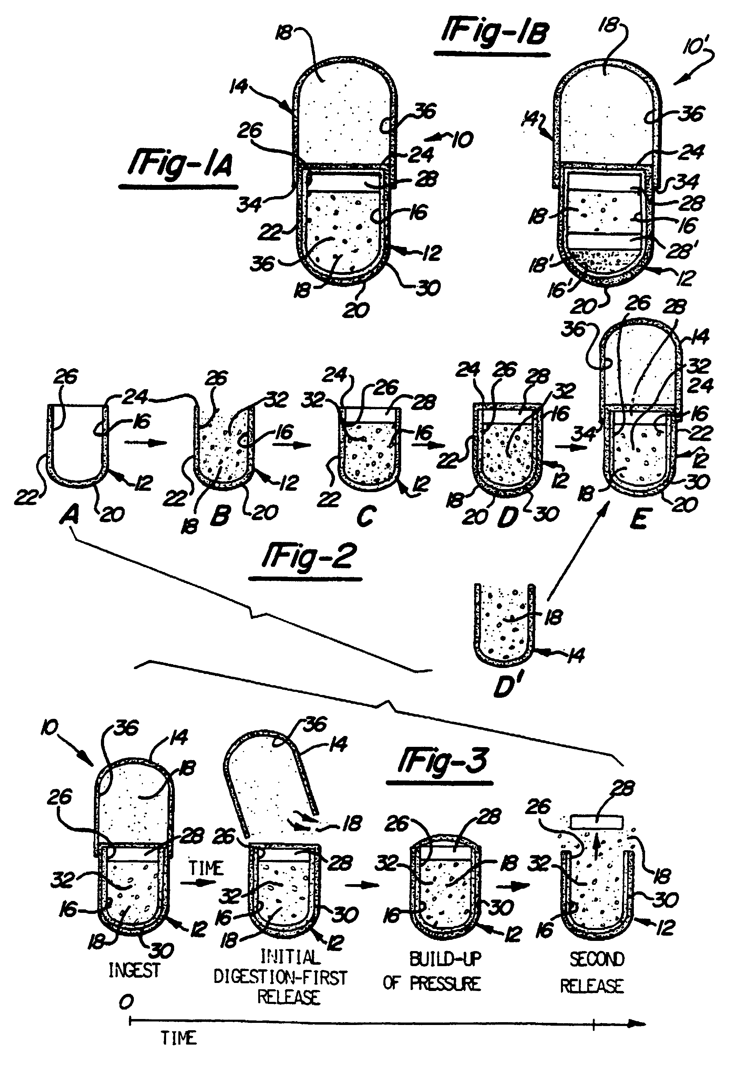

More specifically referring to Figure 1A, the first capsule half 12 includes a closed end portion 20 extending to a substantially annular wall 22 defining a second open end 24. The wall 22 and opening 24 define an internal passageway 26 opening to the external environment thereof. A plug 28 is disposed in the passageway 26 for plugging the opening 24 closed. The plug 28 is releasable from the opening 24 upon the application of pressure from within the inner chamber 16. The invention is characterized by the first capsule 12 including a mechanism reactive with the external environment for increasing the pressure within the inner chamber 16 and forcing the plug 28 out of the passageway 26 to release the drug 18 from the inner chamber 16 and out of the passageway 26.

As shown in Figure 1B, the system can include multiple chambers 16,16' and multiple plugs 28,28'. Each chamber 16,16' includes a mechanism reactive with the external environment for first forcing out plug 28 to release the contents of chamber 16 and then sequentially force out the second plug 28' to release the contents of chamber 16'.

With more specific regard to the reactive mechanism, the reactive mechanism can be a pumping mechanism, such as an osmotic means for pumping fluid through the wall of the first capsule half 12 increasing the internal pressure within the inner chamber 16. Accordingly, once the drug delivery system 10 is ingested at a predetermined time, the reactive mechanism will cause a release of the drug 18 from the first capsule half 16 at predetermined time after ingestion. The rate of internal pressure increase results in the release, timing of the rate being controlled by means described below.

For example, to create the osmotic pump of the present invention, the first capsule half 12 includes a membrane film 30 disposed thereover and over the plug 28 for allowing fluid to pass into the inner chamber 16 as a result of an osmotic pressure gradient therethrough. The osmotic pump further includes an osmotic agent 32 disposed within the inner chamber 16 for creating an osmotic pressure gradient across the membrane film 30 and capsule wall when disposed in the fluid of the external environment.

The open end 34 of the second substantially cupped shaped capsule 14 is seated over and in mating engagement with the open end 24 of the first capsule half 12. The second capsule half 14 includes an inner chamber 36 containing drug 18 therein. The second capsule half 14 is releasably connected to the first capsule half 12 so as to release upon ingestion of the capsule thereby providing an immediate release of drug 18 followed after a predetermined time by the pulse release of the drug 18 from the second capsule half 12.

The capsule halves 12,14 can be made from various materials, preferably water containing gelatins.

The plug 28 can be made from various materials which can, in a plug shape, form a friction fit within the passageway 26 of the first capsule half 12. Examples of plug materials are bees' wax and synthetic bees' wax, carnauba wax, partial glycerides and polyethylene glycol (PEG), fatty esters, glyceryl stearate, palmitosterate, paraffin wax, and white wax.

Various osmotic agents can be used with the present invention. Agents such as lactose, sorbitol and mannitol can be used.

Various film materials can be used for forming the membrane film 30. Examples of composition for forming the film materials are cellulose acetate (all grades), cellulose acetate butyrate (all grades), and combinations of the above. Also, ethylcellulose can be used.

Figure 2 schematically illustrates the method of making the drug delivery system 10 in accordance with the present invention. Step A in Figure 2 shows the first capsule half 12 being empty. Step B shows the capsule half being filled with osmotic reagent 32 and drug 18. As stated above, the drug 18 per se could be the osmotic agent. As shown in Step C, the open end 24 of the first capsule half 12 is plugged with the plug member 28. Step D shows the water permeable film 30 being disposed over the capsule 12 and plug 28. Step D' shows the filling of the second capsule half 14 with the drug 18. Finally, Step E shows the mounting of the open end 34 of the second capsule half 14 over the plugged end 24 of the first capsule half 12.

Of course, many of the steps shown in Figure 2 can be accomplished by various filling, plugging, and coating methods. The capsule was made by the following specific method. Also, multichambered systems can be made by repeating the filling and coating steps.

Weighed citric acid, anhydrous, USP, was disposed in a mortar and ground thoroughly to a fine powder. Anhydrous lactose, USP, microcrystalline cellulose, NF, sorbitol, NF, Croscarmellos sodium, NF, were added to the mortar containing the citric acid, anhydrous, USP and mixed well. The captopril, USP was added to the mortar containing the excipients of the previous step and mixed thoroughly. The magnesium stearate, BP was added to the mortar and stirred gently. Homogeneity of the mixture was checked from three spots in the mortar taking one gram sample. A 98.4% yield was obtained.

A number zero hard gelatin two piece capsule was filled with 350 mg + 1.5 mg of the fill mix or adjusted to give a potency of 67 mg based on the assay result from the previous step. A plurality of capsules were filled.

Gelucire 50/02 was melted using a water bath to a constant temperature of 60°C + 5°C. 120 mg of the melted gelucire 50/20 + 20 mg was filled into each capsule or five drops of the Gelucire was dispensed using a transfer pipette into each capsule. The capsules were allowed to sit until the gelucire sufficiently solidified. The specific weight (amount) of Gelucire or other plug material can be varied. The capsules were weighed and then placed in a 6 inch diameter coating pan. Rotation of the coating pan was started and adjusted to a speed of 30 rpm + 5 rpm. Using a Sigma Glass Spray Unit, the bottle was filled with 225 ml + 25 ml coating solution. A spray top was fitted on the bottle and tightly capped. A suitable spray pattern was obtained using a compressed air unit by adjusting the air flow and the capsules were sprayed in the pan for 60 seconds. The capsules were allowed to turn in the pan with a stream of compressed air blowing into the pan for 60 seconds. The weight gain of the capsules was calculated as follows:

$$\% \text{ gain} = \frac{\text{coated weight - uncoated weight}}{\text{uncoated weight x 100}}$$

Thusly, when referring to coating thickness, percent coat is referenced, that meaning the percent gain in weight of the capsule coated by the membrane film. The greater the percent gain, the thicker the coating on the capsule.

To make the final capsules, 66 mg + 1 mg of the captopril immediate release blend (50%) was disposed into the cap of the size number zero hard gel and mounted onto the capsule previously referred to. The cap was placed on the body taking care not to lose any of the material in the cap or to disrupt the coating on the capsule body. These fine finished capsules were stored in polyethylene bags until tested as described below.

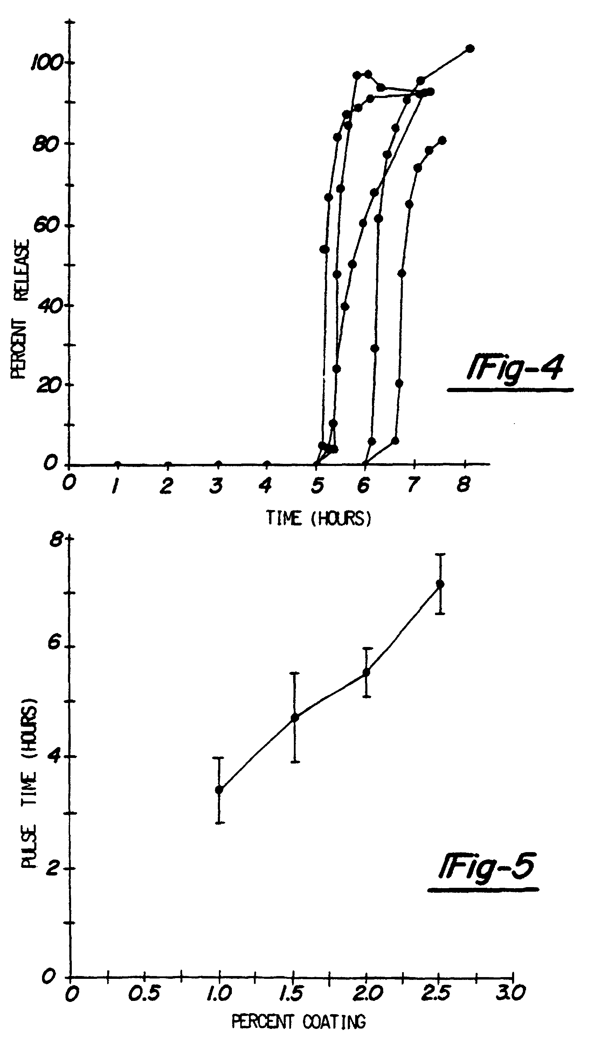

Figure 4 shows the effective ability of the capsules made in accordance with the present invention to generate a pulse release. First capsule halves 12 made in accordance with the method previously described were tested in vitro for ability to create an osmotic pressure therein to force the release of the plug member 23 and thereby release captopril therefrom. The method consisted of the steps of disposing capsule halves coated as previously described in 28 ml of pH 6.5 buffer solution at 37°C. Samples were initially taken once every hour. At the 5 hour time period, samples were taken every 10 minutes.

As shown in Figure 4, there is absolutely no release from the capsules during the first 5 hours of testing. There was an immediate pulsatile release from the capsules beginning at 5 and 6 hours. Accordingly, capsules made in accordance with the present invention have the capacity to pulse release.

Several variables were evaluated with regard to the manufacturing techniques to determine their effect on pulse time.

It has been shown that plug variables such as the effect of the hydrophilic/lypophilic balance HLB of the plug on pulse time was tested. The HLB values were varied by varying the components used to make the plug. For example, different waxes have different HLB values. By combining different waxes, the HLB value of the resulting plug is varied. Additionally, the temperature of the plug material, such as gelucire, prior to filling also effects pulse time.

The effect of coating variables such as spray rate solids content and plasticizer content of the coating have also been determined. It was found that the variables tested were also affected by the percent coating, that is, the weight percent of the coating as compared to the weight of the remainder of the capsule.

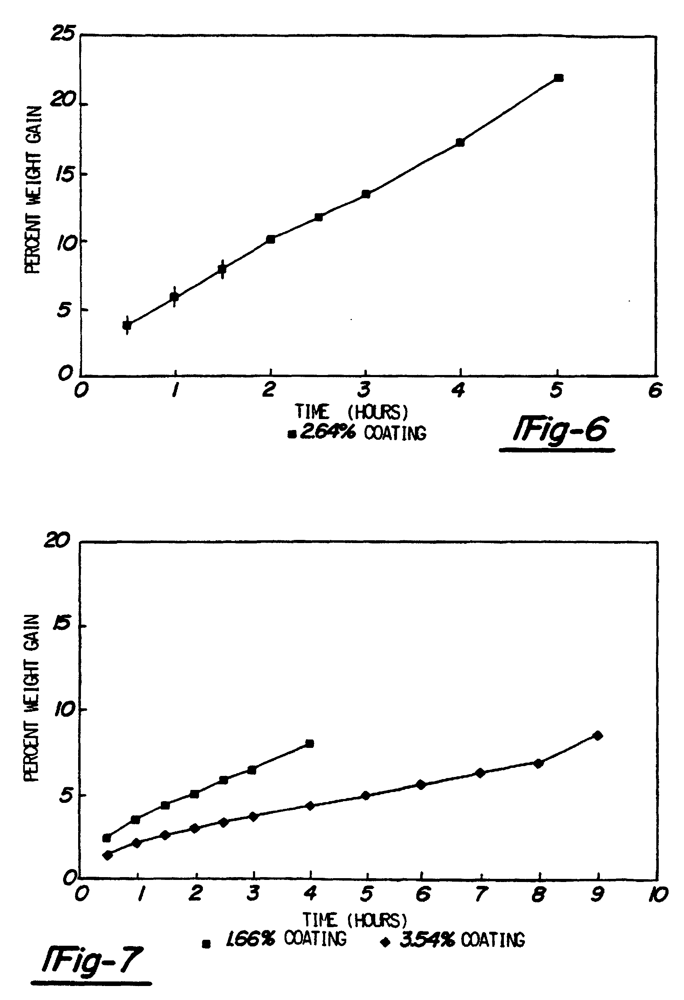

A more detailed analysis of the average pulse time as a function of percent coating is shown in Figure 5. Figure 5 shows an almost linear relationship between increased percent coating and pulse time. Thusly, one method of controlling the predetermined time of release is by changing the percent coating of the capsule. As in the prior experiments, this experiment was conducted by coating capsules as described above, determining their percent coating and the placing the capsules as batches based on their percent coating in 20 ml of buffer, 6.5 pH at 37°C. Captopril release was monitored by high pressure liquid chromotography.

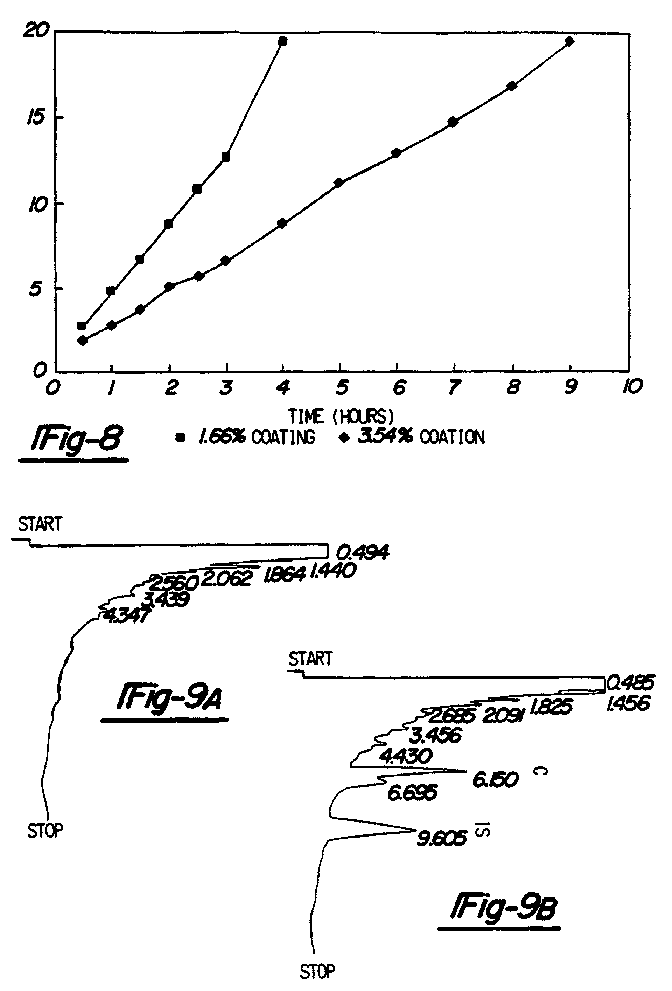

Further studies were conducted on the effect of various osmotic agents as they effect water uptake within capsules. To perform these experiments, capsules as made above but filled with lactose, and lactose/sorbitol filled capsules were disposed in 20 ml of buffer, pH 6.5, at 37°C. Six of each capsule type were placed in buffer, the lactose filled capsules and the lactose/sorbitol filled capsules having either a 1.66 weight percent coating or 3.54 weight percent coating. After the periods of time indicated in Figures 6-8, the weight of the capsule was determined and percent weight gain was determined as showing comparative rates of the osmotic pressure gradients created by the various agents within the capsules.

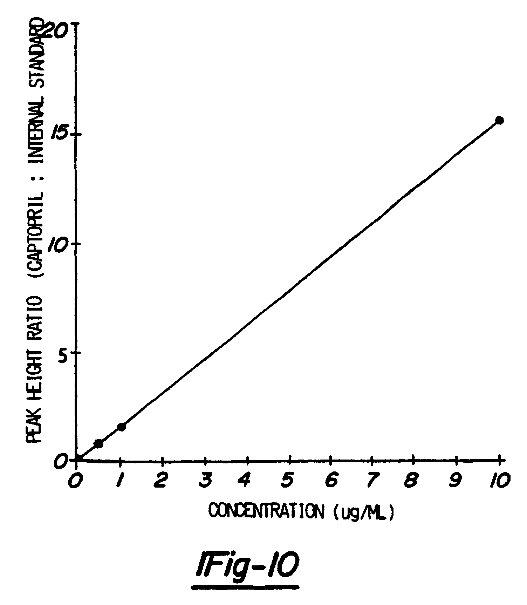

Figure 6 shows an almost linear weight percent gain over time of the Captopril capsules, containing both lactose and sorbitol therein as discussed above. Figure 7 shows a comparative decrease in weight percent gain over time in the capsules containing only lactose. The expected increase in rate is shown with capsules having the thinner coating of 1.66%. A similar phenomenon is shown with the lactose/sorbitol filled capsules, except that these capsules had a significant increase in rate compared to capsules containing lactose alone.

In view of the above data, the rate of the internal pressure gradient increase and the time period to release the plug member can be adjusted and controlled by adjusting the amount and type of osmotic agent within the capsules, as well as adjusting the thickness of the membrane coating.

The present invention further provides a method of delivering a drug to a body, as schematically shown in Figure 3. The steps generally include the ingestion of the drug delivery system 10 as shown in Figure 3. Figure 3 shows the delivery of the drug over a time, the time line being schematically shown at the bottom of the Figure. There is an initial release of the first predetermined amount of drug 18 from the first chamber 36 of the system 10 after ingestion. The first capsule half 12 remains intact within the membrane 30. As the first capsule half 12 travels through the digestive track it is exposed to the fluid therein, there is a pressure buildup within the inner chamber 16 of the system 10 over time. This forces the plug 28 from the passageway 26 at a predetermined time after the ingesting step. Finally, after the predetermined time, the plug 28 is completely forced from the passageway 26 thereby releasing the drug 18 from the inner chamber 16. A multichamber system works in the same manner with a later pulse of drug 18' being released from chamber 16' and plug 28' is forced out of the capsule half 12. As shown by the in vitro experiments above, the rate of osmotic pressure increase of the inner chamber 16 can be controlled by various variables such as the type and amount of osmotic agent as well as the thickness or percent coating of the membrane film 30.

Applicant has conducted bioavailbility studies demonstrating the aforementioned method in vivo.

**MATERIALS**

Capsules, prepared as described above numbered from 3 to 7, were used for bioavailability study. Captopril tablets and powder for oral and intravenous studies were kindly donated by Squibb. Two male beagle dogs, weighing 34 and 30 pounds and two midgut-fistulated female dogs, weighing 47 and 38 pounds respectively, were employed for the bioavailability studies. These dogs were fasted over 15 hours before experiments were began.

Oral study - Four tablets containing 25 mg of Captopril were given to four dogs orally with 20 ml of tap water. Dogs were released from restraining sling for 15 minutes four hours after the experiment was started for urination and a walk. Blood samples (1.2 ml) were collected through the forearm vein, which was catheterized with an 18G catheter (Abbott, Chicago, IL), at 0, 0.25, 0.5, 1, 1.5, 2, 2.5, 3, 3.5, 4, 5, and 6 hours and transferred to test tubes containing 5 mg of each N-ethylmaleimide (NEM) and ethylene diamine tetra acetic acid (EDTA) sodium and stored in a freezer until assay.

Intravenous study - 50 mg of captopril powder was dissolved in 15 ml of saline and filtered through 0.22 um sterilized filter paper right before the infusion was started. For each four dogs, this solution was infused over 15 minutes into the catheterized forearm vein using a Harvard infusion pump. For this intravenous study, blood samples were collected from the other side of the forearm vein at 0, 1, 2, 3, 5, 8, 10, 13, 15, 16, 18, 20, 23, 25, 30, and 40 minutes and at 1, 2, 3, and 4 hours.

The study was duplicated in each dog. The experimental design was the same as the oral study except that the schedule for sample collection was every one hour for 12 to 13 hours. Dogs were released from the sling every four hours for 15 minutes.

GC-EC blood sample assay - All blood samples were assayed using gas chromatography with an electron-captured detector. The GC-EC assay, which was reported earlier by Bathala et al., was slightly modified and tested for linearity, precision, and accuracy. The standard curve was linear over the concentration range studied, with an r value of 0.9999. The detection limit based on a signal-to-noise ratio of 3 were 25 ng/ml. The determination of Captopril was highly reproducible, with a CV of less than 7% for all concentrations examined. The intra-and inter-day variability of the Captopril assay was not significant.

Materials - NEM, hexafluoro-2-propanol, trifluoro acetic anhydride, were reagent grade (Sigma Co., Mo) and used as received. All other chemicals were reagent grade (Fisher Scientific, Chicago) or HPLC grade. Captopril and internal standard, SQ 25761, were obtained from E.R. Squibb & Sons (Princeton). The chromatographic column was capillary, 30 m x 0.53 mm i.d. (1.2 mcl of film thickness), immobilized with 100% dimethyl polysiloxane (Cat. #19656, Alltech Assoc., Chicago IL). Nitrogen and argon-methane (95:5) of the highest available purity (Metro Welding Co., Detroit, MI), were used.

Equipment - Gas chromatography was preformed using HP 5890A (Hewlett Packard) gas chromatograph equipped with a nickel-63 electron-capture detector, 3393A HP integrator, 7673A HP controller, and 7673A HP automatic sampler. All extractions were carried out by shaking the samples on a Tekmar mixer (Janke & Kunkel Co., Funkentstort, West Germany). The N-Evap (Organomation Assoc., Northborough, MA) was used to remove benzene from extracts with a nitrogen stream. The esterification with hexafluoro-2-propanol were performed by incubating in a heating block (Lab-Line Instruments Inc., Melrose Park, IL).

Blood sample assay - After thawing blood samples by sonication, the blood was diluted with distilled water (1:1 by volume). An internal standard (615 ng/ml) was spiked into blood samples and excess NEM and naturally occurring interfering substances were removed by extraction with benzene followed by acidification and extracted with benzene and converted to their hexafluoroisopropyl esters. These were separated by GC-EC. Standard curves from spiked Captopril concentrations of 0.05. 0.5, 1, 10 mcg/ml in blood were prepared for daily working standards. For reproducibility studies, four concentrations for the standard curve were assayed in quadruplicate using the method described.

Data analysis - Area under curves (AUC) of time zero to t and time zero to infinitive by extrapolating the last blood concentration with an elimination rate constant (ke) were evaluated from the oral, intravenous, and technology studies based on the noncompartmental analysis. Relative bioavailability of technology capsules were determined comparing to the oral study and normalized by the dose given.

In view of the experimental results, it can be concluded that capsules made in accordance with the present invention provide a pulsatile release of drugs effective in in vitro environments, as well as in vivo. Such a drug delivery system possess great potential for use in providing drugs to the public that have a first pass effect.

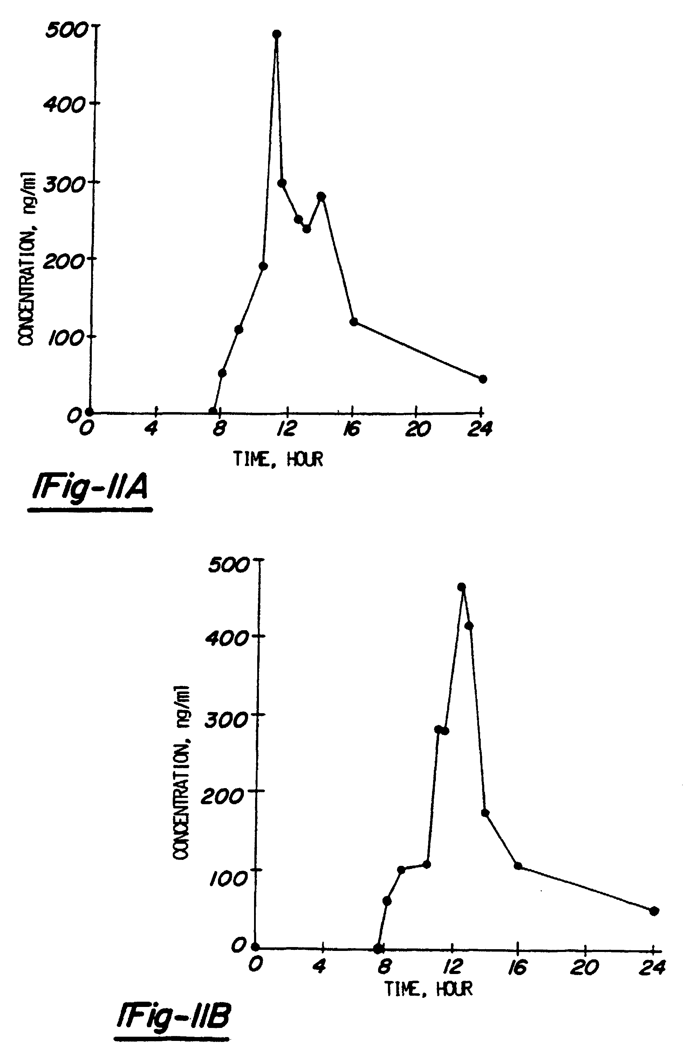

GC-EC assay described above with a slight modification using capillary column, was adequate for the present study. Typical chromatograms from blank blood samples and actual samples from dog studies are shown in Figure 9A and B respectively. The retention times of the derivatized captopril and internal standard were about 6.2 and 9.6 minutes, respectively. These retention times are different from those reported earlier by Bathala et al. This is probably due to the alteration in instrumentations. No interfering peaks were observed in the extracts of the blank dog blood. The derivatives of captopril and of internal standard were stable over one month (testing period) at room temperature.

A plot of chromatographic peak height ratio versus concentration was linear for captopril from 0.05 to 1 mcg/ml (Figure 10). The correlation coefficient and the y-intercept for the straight lines were 0.999 and 0.003, respectively. The average coefficient of variation (CV) for all the concentrations examined was +7%. The linearity and reproducibility of the GC-EC method in dog blood by Bathala et al., was demonstrated by 4 consecutive calibration curves in Figure 2.

Figures 11A and 11B show the results of two dog studies wherein capsules made in accordance with the present invention were ingested. The capsules contained captopril and were made as discussed above. Blood samples were analyzed at the times indicated. There was no release of drug prior to the eight hour time point followed by a pulse or peak of drug. The pulse was a well defined peak. Accordingly, the present invention has been shown to function in vitro as well as in vivo.

## Claims

**1.** A drug delivery system (10), comprising:

a first container (12) including at least one inner chamber (16) for containing a drug (18) and an additional osmotic agent therein and having a passageway (26) opening to an external environment thereof, said container permitting entry therethrough of at least one component of the external environment into said chamber, membrane means (30) disposed over the first container for allowing fluid to pass into the inner chamber when placed in the external environment, and plug means (28) disposed in said passageway (26) for plugging said opening (24) closed, said plug means (28) being releasable from said opening (24) upon the application of pressure from within said inner chamber (16) by the action of pressure reactive means, wherein said pressure reactive means of said first container (12) is osmotic pump means for osmotically pumping fluid through said first container (12), said osmotic pump means includes the drug and the additional osmotic agent (32) disposed within said inner chamber (16) for creating an osmotic pressure gradient across said membrane means (30) when said first container (12) is disposed in the fluid of the external environment, so as to increase the internal pressure within said inner chamber (16), thereby forcing said plug means (28) out of said passageway (26) to release said drug (18) from said inner chamber (16) and out of said passageway (26).

2.  A system according to claim 1, wherein said additional osmotic agent is lactose, sorbitol or mannitol.

3.  A system according to claim 1 or 2, wherein said osmotic means includes pump control means for controlling the rate of osmotic pressure increase within said chamber and the time at which said plug means is released from said passageway.

4.  A system according to any preceding claim, wherein said membrane means is a film selected from cellulose acetate, cellulose acetate butyrate, combinations of cellulose acetate and cellulose acetate butyrate, and ethyl cellulose.

5.  A system according to any preceding claim, wherein said pump control means includes a predetermined thickness of said membrane means, said thickness of said membrane means being inversely related to the rate of travel of said plug means.

6.  A system according to any preceding claim, wherein said plug means (28) consists of a wax material.

7.  A system according to claim 6, wherein the wax material is bees' wax, synthetic bees' wax, carnauba wax, partial glycerides and polyethylene glycol fatty esters, glyceryl stearate, palmitostearate, paraffin wax, or white wax.

8.  A system according to any preceding claim, wherein said first container includes a first substantially cup-shaped water porous capsule half (12) having an open end (24) and a closed end (20) and a wall (22) extending therebetween, said wall (22) defining said passageway (26) about said open end (24), said plug means (28) including a plug member (28) disposed within said open end (24) and in friction engagement with said wall (22) thereabout, said membrane means including a water porous film (30) disposed over said capsule half (12) and plug member (28).

9.  A system according to any preceding claim, wherein the system includes multiple chambers and multiple plugs.

10. A system according to claim 8 or 9, which includes a second substantially cup-shaped capsule (14) having an open portion (34) releasably mounted about said plugged opening (24) of said first capsule (12) and defining a second chamber (36) for containing drug (18) therewithin, said second capsule (14) releasing a predetermined amount of drug (18) upon initial ingestion thereof and said first capsule (12) releasing a second predetermined amount of drug (18) at a predetermined period thereafter, resulting in an initial release of drug (18) upon ingestion and a later independent release of drug (18) at a time dependent upon said pump control means.

11. A system according to any of claims 1 to 7, further including a second container (14) having an open end (34) releasably mounted on said first container (12) and closed thereby for forming a closed second chamber (36) therewithin for containing drug (18), said second container (14) being releasable from said first container (12) upon ingestion to immediately release drug (18), said second container (14) releasing the other portion of drug (18) at a predetermined time thereafter; and/or wherein said first container (12) includes more than one inner chamber (16,16') for containing drug in each chamber, plug means (28,28') being disposed in said passageway (26) for plugging and separating each of said inner chambers, (16,16') and pressure creating means in each of said inner chambers.

12. A method of making a drug delivery system (10) including the steps of:

    filling a first capsule half (12) with a drug and an osmotic agent, the capsule being water permeable;

plugging an open-end (24) of the first capsule half (12);

disposing a water permeable film (30) over the first capsule half (12) and plug (28);

filling a second capsule half (14) with a drug (18);

and releasably mounting an open end (34) of the second capsule half (14) over the plugged end (24) of the first capsule half (12).

13. A method according to claim 12, wherein the permeable film is applied by spray coating.

## Patentansprüche

1. Arzneistoffabgabensystem (10), umfassend:

einen ersten Behälter (12), umfassend zumindest eine Innenkammer (16) zum Beinhalten eines Arzneistoffes (18) und ein zusätzliches osmotisches Agens, und mit einem Durchgang (26), der zu einer äußeren Umgebung des Behälters geöffnet ist, wobei der Behälter dadurch den Eintritt zumindest einer Komponente der äußeren Umgebung in die Kammer gestattet,

ein Membranmittel (30), das um den ersten Behälter herum angeardnet ist, um den Eintritt eines Fluids in die Innenkammer zu ermöglichen, wenn es in die äußere Umgebung gebracht wird, und

ein Verschlussmittel (28), das in dem Durchgang (26) angeordnet ist zum Verschließen der Öffnung (24), wobei das Verschlussmittel (28) aus der Öffnung (24) freisetzbar ist auf Applikation von Druck aus der Innenkammer (16) hin, der durch die Wirkung eines druckreaktiven Mittels hervorgerufen wird, wobei das druckreaktive Mittel des ersten Behälters (12) ein osmotisches Pumpmittel zum osmotischen Pumpen von Fluid durch den ersten Behälter (12) ist, und das osmotische Pumpmittel den Arznelstoff und das zusätzliche osmotische Agens (32) umfasst, das angeordnet ist in der Innenkammer (16) zum Erzeugen eines osmotischen Druckradienten über das Membranmittel (30), wenn der erste Behälter (12) In dem Fluid der äußeren Umgebung angeordnet wird, um den Innendruck in der innenkammer (16) zu erhöhen und dadurch das Verschlussmittel (28) aus dem Durchgang (28) zu treiben und den Arzneistoff (18) aus der innenkammer (18) und durch den Durchgang (26) freizusetzen.

2. System nach Anspruch 1, wobei das zusätzliche osmotische Agens Lactose, Sorbitol oder Mannitol ist.

3. System nach einem der Ansprüche 1 oder 2, wobei die osmotischen Mittel Pumpsteuermittel umfassen zum Steuern der Rate des osmotischen Druckanstiegs in der Kammer und des Zeitpunkts, zu dem das Verschlussmittel aus dem Durchgang freigesetzt wird.

4. System nach einem der vorherigen Ansprüche, wobei das Membranmittel ein Film ist ausgewählt aus Celluloseacetat, Celluloseacetat-Butyrat, Kombinationen von Celluloseacetat und Celluloseacetat-Butyrat, und Ethylcellulose.

5. System nach einem der vorherigen Ansprüche, wobei das Pumpsteuermittel eine vorgewählte Dicke des Membranmittels umfasst, wobei die Dicke des Membranmittels in umgekehrter Beziehung zur Bewegungsrate der Verschlussmittel steht.

6. System nach einem der vorherigen Ansprüche. wobei das Verschlussmittel (28) aus einem Wachsmaterial besteht.

7. System nach Anspruch 6, wobei das Wachsmaterial Bienenwachs, synthetisches Bienenwachs, Camaubawachs, partielle Glyceride und Polyethylenglycol-Fettsäureester, Glycerylstearat, Palmitostearat, Paraffinwachs oder weißes Wachs (white wax) ist.

8. System nach einem der vorherigen Ansprüche, wobei der erste Behälter eine im Wesentlichen tassenförmige, wasserdurchlässige Kapselhälfte (12) mit einem offenen Ende (24) und einem geschlossenen Ende (20) und einer sich dazwischen erstreckenden Wand (22) umfasst, wobei die Wand (22) den Durchgang (26) um das offene Ende (24) bildet, und wobei das Verschlussmittel (28) ein Verschlusselement (28) umfasst, das in dem offenen Ende (24) angeordnet ist und darin in Reibe-Eingriff mit der Wand (22) steht, und wobei das Membranmittel einen wasserdurchlässigen Film (30) umfasst, der um die Kapselhälfte (12) und das Verschlusselement (28) herum angeordnet ist

9. System nach einem der vorherigen Ansprüche, wobei das System mehrere Kammern und mehrere Verschlüsse umfasst.

10. System nach einem der Ansprüche 8 oder 9, umfassend eine zweite im Wesentlichen tassenförmige Kapsel (14) mit einem offenen Abschnitt (34), freisetzbar an der verschlossenen Öffnung (24) der ersten Kapsel (12) angebracht und eine zweite Kammer (36) zum Beinhalten des Arzneistoffes (18) darin bildend, wobei die zweite Kapsel (14) eine vorbestimmte Menge des Arzneistoffes (18) bei ihrer ersten Einnahme freisetzt und die erste Kapsel (12) eine zweite vorbestimmte Menge des Arzneistoffes (18) zu einer vorbestimmten späteren Zeit freisetzt, sodass ein anfängliches Freisetzen des Arzneistoffes (18) nach der Einnahme und eine spätere unabhängige Freisetzung des Arzneistoffes (18) zu einer Zeit, die von den Pumpsteuermitteln abhängt, bewirkt wind.

11. System nach einem der Ansprüche 1 bis 7, ferner umfassend einen zweiten Behälter (14) mit einem offenen Ende (34), lösbar angebracht auf dem ersten Behälter (12) und dadurch geschlossen, zum Bilden einer geschlossenen zweiten Kammer (36) darin zum Beinhalten des Arzneistoffes (18), wobei der zweite Behälter (14) freisetzbar vom ersten Behälter (12) bei Einnahme ist, um sofort den Arzneistoff (18) freizusetzen, wobei der zweite Behälter (14) den anderen Anteil des Arzneistoffes (18) zu einer vorbestimmten späteren Zeit freisetzt; und/oder
wenn der erste Behälter (12) mehr als eine Innenkammer (16, 16') umfasst zum Beinhalten eines Arzneistoffes in jeder Kammer, Verschlussmittel (28, 28') in den Durchgang (28) zum Verschließen und Abtrennen jeder der Innenkammem (16, 16'), und druckerzeugende Mittel in jeder der Innenkammem.

12. Verfahren zum Herstellen eines Arzneistoffabgabesystems (10), umfassend die Schritte:

Befüllen einer ersten Kapselhälfte (12) mit einem Arzneistoff und einem osmotischen Agens, wobei die Kapsel wasserdurchlässig ist;

Verschließen eines offenen Endes (24) der ersten Kapselhälfte (12);

Anordnen eines wasserdurchlässigen Films (30) auf der ersten Kapselhälfte (12) und dem Verschluss (28);

Befüllen einer zweiten Kapselhälfte (14) mit einem Arznelstoff (18);

und freisetzbar Anbringen eines offenen Endes (34) der zweiten Kapselhälfte (14) über dem verschlossenen Ende (24) der ersten Kapselhälfte (12).

13. Verfahren nach Anspruch 12, wobei der durchlässige Film durch Sprühauftragung appliziert wird.

**Revendications**

1. Système d'apport médicamenteux (10) comprenant :

un premier récipient (12) comportant au moins une chambre interne (16) destinée à recevoir un médicament (18) ct un agent osmotique supplémentaire et comportant un passage (26) s'ouvrant vers un environnement externe à celui-ci, ledit récipient permettant l'entrée par celui-ci d'au moins un composant de l'environnement externe dans ladite chambre, un moyen à membrane (30) disposé sur le premier récipient pour permettre le passage de fluide dans la chambre interne lorsqu'il est placé dans l'environnement externe, et un moyen d'obturation (28) disposé dans ledit passage (26) pour boucher ladite ouverture (24) en position de fermeture, ledit moyen d'obturation (28) pouvant être libéré de ladite ouverture (34) en appliquant une pression depuis ladite chambre interne (16) par l'action d'un moyen de réaction à la pression, où ledit moyen de réaction à la pression dudit premier récipient (12) est un moyen à pompe osmotique destiné à pomper par osmose du fluide à travers ledit premier récipient (12), ledit moyen à pompe osmotique comprenant le médicament et l'agent osmotique supplémentaire (32) disposés au sein de ladite chambre interne (16) pour créer un gradient de pression osmotique à travers ledit moyen à membrane (30) lorsque ledit premier récipient (12) est disposé dans le fluide de l'environnement externe, de façon à accroître la pression interne au sein de ladite chambre interne (16) et ainsi à forcer ledit moyen d'obturation (28) en dehors dudit passage (26) pour libérer ledit médicament (18) de ladite chambre interne (16) et dudit passage (26).

2. Système selon la revendication 1, dans lequel ledit agent osmotique supplémentaire est du lactose, du sorbitol ou du mannitol.

3. Système selon la revendicatipn 1 ou 2, dans lequel ledit moyen osmotique comprend un moyen de commande à pompe pour contrôler le taux d'augmentation de la pression osmotiyue au sein de ladite chambre et le moment auquel ledit moyen d'obturation est libéré dudit passage.

4. Système selon l'une quelconque des revendications précédentes, dans lequel ledit moyen à membrane est un film sélectionné parmi l'acétate de cellulose, l'acétate-butyrate de cellulose, des combinaisons d'acétate de cellulose et d'acétate-butyrate de cellulose, et l'éthylcellulose.

**5.** Système selon l'une quelconque des revendications précédentes, dans leguel ledit moyen de commande à pompe comprend une épaisseur déterminée du dit moyen à membrane, ladite épaisseur de ladite membrane étant inversement liée à la vitesse de déplacement du dit moyen d'obturation.

**6.** Système selon l'une quelconque des revendications précédentes, dans lequel ledit moyen d'obturation (28) se compose d'un matérisu cireux.

**7.** Système salon la revendication 6, dans lequel le matériau cireux est de la cire d'abeille, de la cire d'abeille synthétique, de la cire de carnauba, des glycérides partials et des esters gras de polyéthylèneglycol, du stéarate de glycéryle, du palmitostéarate, de la cire de paraffine ou de la cire blanche.

**8.** Système selon l'une quelconque des revendications précédentes, dans lequel ledit premier récipient comprend une première demi-gélule (12) poreuse à l'eau et substantiellement en forme de coupelle, présentant une extremité ouverte (24) et une extrémité fermée (20), ainsi qu'une paroi (22) s'étendant entre les deux, ladite paroi (22) définissant ledit passage (26) autour de ladite extrémité ouverte (24), ledit moyen d'obturation (28) comprenant un élément d'obturation (28) disposé au sein de ladite extrémité ouverte (24) et s'engageant par frottement avec ladite paroi (22), ledit moyen à membrane comprenant un film poreux à l'eau (30) disposé sur ladite demi-gélule (12) et l'élément d'obturation (28).

**9.** Système selon l'une quelconque des revendications précédentes, dans lequel le système comprend plusieurs chambres et plusieurs obturateurs.

**10.** Système selon la revendication 8 ou 9, qui comprend une seconde gélule (14) substantiellement en forme de coupelle et présentant une portion ouverte (34) montée de façon libérable autour de ladite ouverture obturée (24) de ladite première gélule (12) et définissant une seconde chambre (36) destinée à recevoir un médicament (18) à l'intérieur, ladite seconde gélule (14) libérant une quantité prédéfinie de médicament (18) lors de l'ingestion initiale de celle-ci, et ladite première gélule (12) libérant une seconde quantité prédéfinie de médicament (18) à un moment ultérieur prédéfini, ce qui entraîne une libération initiale de médicament (18) lors de l'ingestion et une libération indépendante ultérieure de médicament (18) à un moment qui dépend dudit moyen de commande à pompe.

**11.** Système selon l'une quelconque des revendications I à 7, comprenant en outre un second récipient (14) présentant une extrémité ouverte (34) montée de façon libérable sur ledit premier récipient (12) et fermée par celui-ci pour former une seconde chambre fermée (36) à l'intérieur destinée à recevoir un médicament (18), ledit second récipient (14) pouvant être libéré dudit premier récipient (12) lors de l'ingestion pour libérer immédiatement le médicament (18), ledit second récipient (14) libérant l'autre portion de médicament (18) à un moment ultérieur prédéfini ; et/ou dans lequel ledit premier récipient (12) comporte plus d'une chambre interne (16, 16') destinée chacune à recevoir un médicament, des moyens d'obturation (28, 28') étant disposés dans ledit passage (26) pour obturer et séparer chacune desdites chambres internes (16, 16'), ainsi que des moyens de création de pression dans chacune desdites chambres internes.

**12.** Procédé de fabrication d'un système de délivrance d'un médicament (10) comprenant les étapes suivantes :

remplir une première demi-gélule (12) d'un médicament et d'un agent osmotique, la capsule étant perméable à l'eau ;
obturer une extrémité ouverte (24) de la première demi-gélule (12);
disposer un film perméable à l'eau (30) sur la première demi-gélule (12) et l'obturateur (28) ;
remplir une seconde demi-gélule (14) d'un médicament (18) ;
et monter de façon libérablc une extrémité ouverte (34) de la scconde demi-gélule (14) sur l'extrémité obturée (24) de la première demi-gélule (12).

**13.** Procédé selon la revendication 12, dans lequel le film perméable est appliqué par couchage par pulvérisation.

*Fig-1B*

*Fig-1A*

*Fig-2*

*Fig-3*

INGEST

INITIAL
DIGESTION-FIRST
RELEASE

BUILD-UP
OF PRESSURE

SECOND
RELEASE

TIME

Fig-4

Fig-5

_ 2.64% COATING

_Fig-6_

_ 1.66% COATING      ♦ 3.54% COATING

_Fig-7_

EP 0 695 174 B1

*Fig-8*  ■ *1.66% COATING*  ♦ *3.54% COATION*

START
0.494
2.560 2.062 1.864 1.440
3.439
4.347

*Fig-9A*

STOP

START
0.485
2.685 2.091 1.825 1.456
3.456
4.430
6.150
6.695
9.605

*Fig-9B*

STOP

14

_IFig-10_

_[Fig-IIA_

_[Fig-IIB_